# EUROPEAN PATENT APPLICATION

(11) **EP 3 973 933 A1**
(43) Date of publication of application: **30.03.2022**
(21) Application number: 20757943.4
(22) Date of filing: 25.06.2020
(51) Int. Cl.: A61F 9/007

(54) **GLAUCOMA DRAIN IMPLANT SYSTEM WITH PRESSURE SENSOR AND VALVE, AND EXTERNAL READING UNIT**

(30) Priority: 26.06.2019 US 201962866969 P
(71) Applicant: Imvalv S.A., Prov. De Santa Fe 2322 (AR); Consejo Nacional de Investigaciones Científicas Y Técnicas (CONICET), Buenos Aires 1425 (AR)
(72) Inventor: GUARNIERI, Fabio Ariel, Sunchales, Prov. De Santa Fe 2322 (AR)
(74) Representative: Bardehle Pagenberg Partnerschaft mbB Patentanwälte Rechtsanwälte
(86) International application number: PCT/IB2020/056038
(87) International publication number: WO 2020/261184

(57) **Abstract**

Glaucoma drainage implant system with intracular pressure sensor (IOP) and microvalve, and external reading unit characterized in that it comprises:
(1) an ocular implant device (1) comprising a main body attached to a cannula (3) with an inlet port (4) which communicates through a microchannel (10) that passes through said cannula with a microchamber of the sensor (11) on which a sensor (or more) (5) is/are located; said microchamber is in fluid communication with a microvalve (2) which regulates the passage of ocular liquid to an outlet port (7), where the microvalve (2) is covered by a plate in said main body, the implant device has also a flat coil (6) to energize the sensor(s) and the microvalve and a microchip (12) for regulation of a microvalve actuator and signal processing and modulation generated by the sensor(s);
(2) an external reading unit (UEL) (13), which acquires and processes signals from the PIO sensor 5) and presents the IOP pressure data on a screen; the UEL (13) mainly consists of two parts: an antenna and a main unit; where the antenna feeds by bursts of RF radio frequency energy to the implant sensor (5) and when the sensor (5) is energized, it returns a signal with information from the IOP, this signal being received by said antenna and sent to the main unit for processing.

## Description

### FIELD OF THE INVENTION

The present invention is included within the field of intraocular implants with a permanent implantable aqueous shunt to reduce intraocular pressure (IOP) in the anterior chamber of the eye where the implant includes a pressure sensor to provide IOP measurement and externally monitor the hydraulic resistance and a microvalve associated with the sensor that allows drainage.

### OBJECT OF THE INVENTION

Glaucoma is a progressive disease that leads to blindness caused by a gradual or sometimes rapid increase in intraocular pressure. Increased intraocular pressure is considered a very important risk factor for contracting blindness and it is estimated that nearly 67 million people suffer from glaucoma worldwide.

The glaucoma drainage implant system with sensors and external reading unit of the present invention is indicated to reduce intraocular pressure in patients with refractory or non-refractory glaucoma. The device includes, for example, a pressure sensor to measure IOP wirelessly to provide the healthcare professional with on-demand information, as well as alert the patient to necessary changes that require medical intervention. This information is used by the medical professional to guide the therapy.

### BACKGROUND OF THE INVENTION

The first passive wireless sensors were proposed in 1967, they used a miniature spiral inductor (L) and a pressuresensitive capacitor (C) to build a resonant sensor that could measure the pressure of the fluid inside the eye (an intraocular pressure sensor). The idea was based on a mechanically adjusted capacitor (varactor), which has been an effective way to tune resonant circuits since the advent of radio. Despite this, wireless capacitive sensing technology has expanded rapidly only in the past two decades, due to the development of microelectromechanical systems (MEMS), nanotechnology, and wireless technology.

In previous years, low-profile passive LC oscillator circuit-based wireless sensors (typically a series of RLC tanks) have been used to measure pressures (Chen P.-J., Rodger DC, Saati S., Humayun MS, Tai Y.-C. Implantable parylene-based wireless intraocular pressure sensor. Proceedings of the IEEE 21st International Conference on Micro Electro Mechanical Systems, 2008 (MEMS 2008); Tucson, AZ, USA. 13-17 January 2008; pp. 58-61). The working principle of these passive LC sensors is typically based on detecting variations of concomitant resonance frequencies, where the quantity to be measured waves out the capacitive or inductive elements of the sensor. This could occur, for example, through mechanical deflection of electrodes. In general, the reading of data from the wireless sensor depends on mutual inductive coupling, and the sensor information is encoded in the reflection coefficient. Such a telemetric sensor system can be modeled using a simple equivalent circuit model, in which the compact sensors are represented by a series of resonant RLC tanks, where a resistance R takes into account the dissipative power of the sensor.

Although there has been continued progress in mechanized micro- and nano-sensors in recent years, the basics of data reading telemetry techniques remain essentially unchanged from the present invention. However, improvements in detection limits are often hindered for available levels of Q factor, sensing resolution, and sensitivity relative to resonance spectral variation in response to variations in physical properties to be measured. In particular, modern LC microsensors based on thin-film resonators or actuators, usually have a low modal Q factor, due to relevant power dissipation caused by skin effect, Eddy currents, and surrounding environments with electrical loss (such as biological tissues). Sharp narrowband reflection tilt has long been a coveted goal for inductive sensor telemetry, since it could achieve superior detection and sound fastness. However, although many devices were made using impedance or network analyzers, they were not portable for use in patients.

Attempts to obtain portable measurement techniques using wireless LC pressure sensor tanks, DDS with phase detector were reported by Hong and Bao, H Zhang, some researchers have reported front circuit concepts for wireless datareading sensor systems. These reading circuits are designed to demodulate changes in impedance parameters of the terminal reading antenna due to mutual coupling.

Nopper et al. (Nopper R., Has R., Reindl L. A Wireless Sensor Readout System - Circuit Concept, Simulation, and Accuracy. IEEE Trans. Instrum. Meas. 2011; 60: 2976-2983) have extracted the resonance frequency signal from a sensor at a distance by multiplication of the signal source of the reading antenna terminal and the modulation signal produced by mutual coupling, thereby filtering and demodulating the actual part of the impedance of a reading antenna terminal; however, the accuracy of the reading circuit was strongly influenced by the Q factor of the remote sensor. Coosemans et al. (Coosemans J., Catrysse M., Puers R. A readout circuit for an intraocular pressure sensor. Sens. Actuator A Phys. 2004; 110: 432-438) studied reading circuits based on a voltage controlled oscillator (VCO), which generated a frequency selection circuit to drive the read antenna coil.

The VCO output signal would produce a mutation point in the resonant frequency of the remote sensor in a sweep period. However, the results were not satisfactory.

Salpavaara et al. (Salpavaara T., Verho J., Kumpulainenm P., Lekkala J. Readout methods for an inductively coupled resonance sensor used in pressure garment application. Sens. Actuators A: Phys. 2011; 172: 109-116) studied the detection circuit hardware phase difference, with compensation for resonance frequency changes with remote coupling, and build a system for clothing pressure monitoring.

Bao et al. (Bao K., Chen D., Shi Q., Liu L., Chen J., Li J., Wang J. "A readout circuit for wireless passive LC sensors and its application for gastrointestinal monitoring." Meas. Sci. Technol. 2014; 25 doi: 10.1088/0957-0233/25/8/085104) built a phase difference detection method based on DSP, and amplification of weak signals over a large distance through a differential mode; however, the working frequency band is very low, less than 1 MHz. The sensitive components and reading circuits in the mutual coupling systems mentioned above were designed to be applied in more than a safe environment. In other words, when the sensors are operated, only the sensor's sensory capacitance would change, but the sensor parameters would remain the same.

H. Zhang et al. (Zhang H, Hong Y, Liang T, et al. Phase interrogation used for a wireless passive pressure sensor in an 800 °C high-temperature environment. Sensors (Basel). 2015; 15(2): 2548-2564. Published 2015 Jan 23. doi:10.3390/s150202548) presented a complete wireless passive measurement system and analyzed the impedance parameters of the wireless passive mutual coupling model and the impedance phase of terminal variation of the read antenna with the Q factor of the LC resonant sensor under high temperature conditions. Other researchers proposed to calculate the sensor output through analytical formulas, the system parameters and part of the real impedance, which is always measured at a fixed frequency. This allows for easier reading electronics.

The document "MEMS-based wireless intraocular presure sensor" to R. Blue and D. Uttamchandany published in Woodhead Publishing Limited, 2013, shows the application of MEMs devices for wireless eye pressure sensors.

Patent U.S. 6,168,575 to Soltanpour et al. entitled "Method and apparatus for controlling eye pressure", describes a small pump from 5 to 15 mm in length which is implanted in the eye to remove excess of fluid that can be adjusted manually or automatically. The control is performed by a pressure sensor connected to a microprocessor, the sensor being arranged externally to the eye, wherein said patent discusses the disadvantage of automatic adjustment as the complications related to muscle hypotonia [paragraph 0009] .

Patent U.S. 6,589,203 describes an implantable ocular device that has a deformation surface made of a material capable of withstanding continuous deformation and a drain tube that has a valve sensitive to pressure variations that limits the flow that circulates through the tube.

Patent U.S. 6,682,500 to Soltanpour et al., refers to "Diaphragm Pump-Based Synthetic Muscle Apparatus," describing a device made with a diaphragm pump made from a synthetic polymer of a metal compound and including a pressure sensor. This device is equipped with two valves, an outlet duct, to regulate the fluid flow in the pump. This reference also allows to implement an inductive coupling to transfer signals between the implant and an external accessory (see paragraph [0010].

Unfortunately, the above mentioned implants have size and biocompatibility problems since they do not allow to meet design requirements.

Less relevant references but that could be of interest in the field are French Patent 2,533,658 directed to valve implant to cure glaucoma and U.S. Patent No. 4,282,882 directed to apparatus for modifying intraocular pressure, U.S. Patent No. 4,402,681 directed to implantable artificial valve to regulate intraocular pressure, U.S. Patent No. 4,585,457 directed to inflatable intraocular lenses, U.S. Patent No. 4,886,488 and U.S. Patent U.S. directed to the method and system of lacrimal drainage of glaucoma, U.S. Patent U.S. 5,454,796 directed to device and method for controlling intraocular fluid pressure, U.S. Pat. 5,520,631; 5,704,907 and 5,433,701 directed to apparatus for reducing eye pressure, U.S. Patent No. 5,454,796 directed to device and method for controlling intraocular fluid pressure, U.S. Pat. 5,520,631, U.S. 5,704,907 and U.S. 6,102,045 directed to methods and apparatus for lowering intraocular pressure, U.S. Patent No. 5,523,808 directed to ophthalmic apparatus provided with an intraocular pressure measurement system, U.S. Patent No. 5,626,559 directed to ophthalmic device to drain excess intraocular fluid, U.S. Patent No. 5,651,782 directed to method and apparatus for implanting a mesh in glaucoma surgery, U.S. Patent No. 5,656,026 directed to in vitro test method of a one-way valve gradient limiting device for draining glaucoma, U.S. Patent No. 5,713,844 directed to a device and method for regulating intraocular pressure, U.S. Patent No. 5,743,868 directed to implantable corneal device for pressure regulation, U.S. Patent No. 5,785,674 directed to a device and method for treating glaucoma, U.S. Patent No. 5,807,302 directed to glaucoma treatment, U.S. Patent No. 5,868,697 directed to intraocular implant, U.S. Patent No. 5,968,058 directed to device and method for implanting an intraocular implant, U.S. Patent No. 6,077,299 directed to a non-invasive implant with adjustable valve to drain aqueous humor in glaucoma, U.S. Patent No. 6,083,161 directed to apparatus and method for improving established eye pressure, U.S. Patent No. 6,113,342 directed to diagnostic method and apparatus for providing effective intraocular pressure based on measurements of the cornea, U.S. Patent No. 6,464,724 directed to a stent device and method of treating glaucoma, U.S. Patent No. 6,468,283 directed to a method for regulating pressure with an ocular implant, U.S. Patent No. 6,510,600 directed to a method for manufacturing a flow-regulated implant, U.S. Patent Nos. 6,558,342 and U.S. 6,730,056 directed to an ocular implant for the treatment of glaucoma and its manufacturing method.

U.S. Patent U.S. 6,939,299 refers to a continuous implantable eye pressure sensor.

None of the prior art documents considered have taken into account the association of valves integrated with the sensor; this combination is not obvious considering the synergy between monitoring and pressure, taking into account valve performance based on other processes that occur such as fibrosis progression and other complications that indirectly affect the sensor pressure measurement.

### SUMMARY OF THE INVENTION

The glaucoma drainage implant system with intracular pressure sensor (IOP) and microvalve, and external reading unit comprises:
(1) an eye implant device (1) comprising a main body attached to a cannula (3) with an inlet port (4) which communicates through a microchannel (10) that passes through said cannula with a microchamber of the sensor (11) on which is a sensor (5); said microchamber is in fluid communication with a microvalve (2) which regulates the passage of ocular liquid to an outlet port (7), where the microvalve (2) is covered by a plate on said main body, the implant device has also a flat coil (6) to energize the sensor and the microvalve and a microchip (12) for regulation of a microvalve actuator and signal processing and modulation generated by the sensor;
(2) an external reading unit (UEL) (13), which acquires and processes signals from the PIO sensor 5) and presents the IOP pressure data on a screen; the UEL (13) mainly consists of two parts: an antenna and a main unit; where the antenna feeds by bursts of RF radio frequency energy to the implant sensor (5) and when the sensor (5) is energized, it returns a signal with information from the IOP, this signal being received by said antenna and sent to the main unit for processing.

In the glaucoma drainage implant system with intracular pressure sensor (IOP) and microvalve, and external reading unit according to the above, the plate (8) of the implant device (1) has at the opposite end of the cannula (3) a fixation hole (9) that allows the fixation of said implant to the sclera of the eye by suture.

### DESCRIPTION OF THE FIGURES

These and other features and details of the invention and the manner in which it can be embodied and practiced should be better understood by the detailed description of the examples not limited to the embodiments of the invention illustrated in the attached figures. Other variations, modifications, adaptations and/or additions can be made without falling outside the spirit and scope of the present invention.

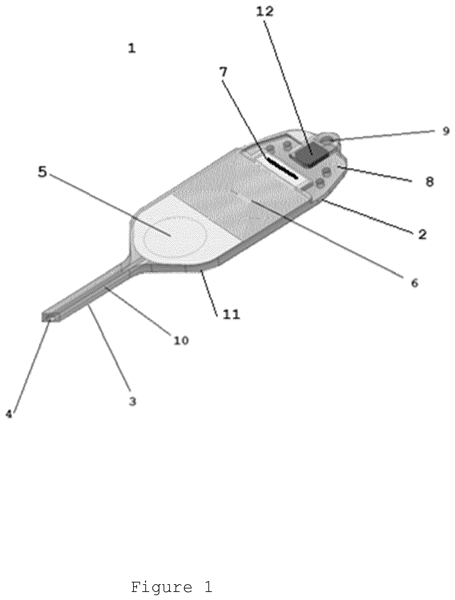

FIG. 1: represents a perspective view from the upper side of the glaucoma drainage implant with a pressure sensor seen from the top of the microvalve where the cannula with the fluid inlet is observed on the left side of the valve, followed by the sensor and the flat coil, an external hole where the fluid is drained, a plate to extend the area of absorption by the conjunctiva that covers the microvalve, and on the plate at the opposite end to the inlet hole of the cannula, a hole to fix the implant to the sclera, the microchip is also observed for the microvalve actuator or signal processing and modulation.

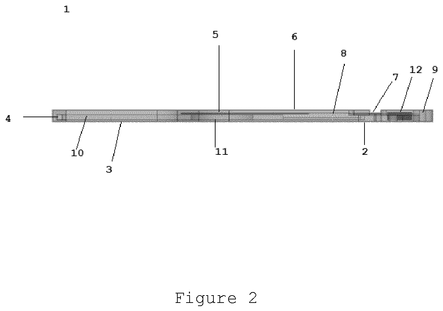

FIG. 2: represents a side view of the pressure sensor glaucoma drainage implant.

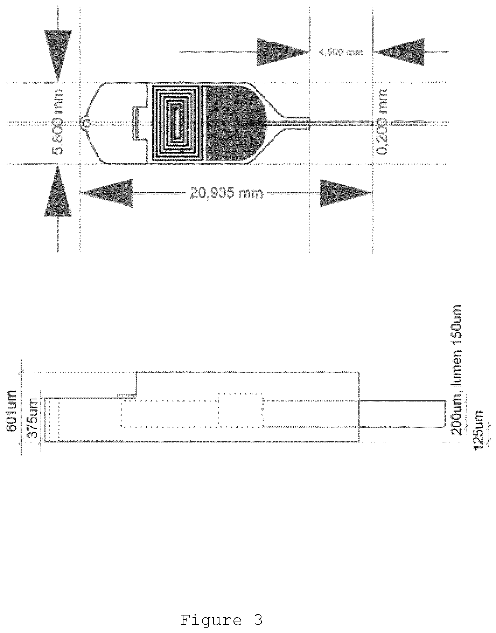

FIG. 3: represents in the drawing above a top view of a variant of the glaucoma drainage implant with pressure sensor with the dimensions in millimeters and in the drawing below a side view of an enlarged profile of said variant of the glaucoma drainage implant with pressure sensor with dimensions in microns.

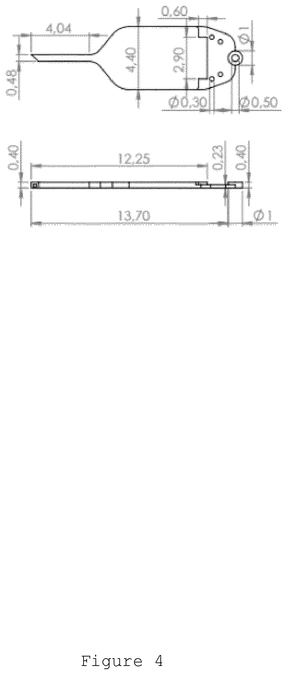

FIG. 4: represents in the drawing above a top view of a variant of the drainage implant for glaucoma with pressure sensor with the dimensions in millimeters and in the lower drawing a side view of said variant of the drainage implant for glaucoma with sensor pressure with dimensions in millimeters.

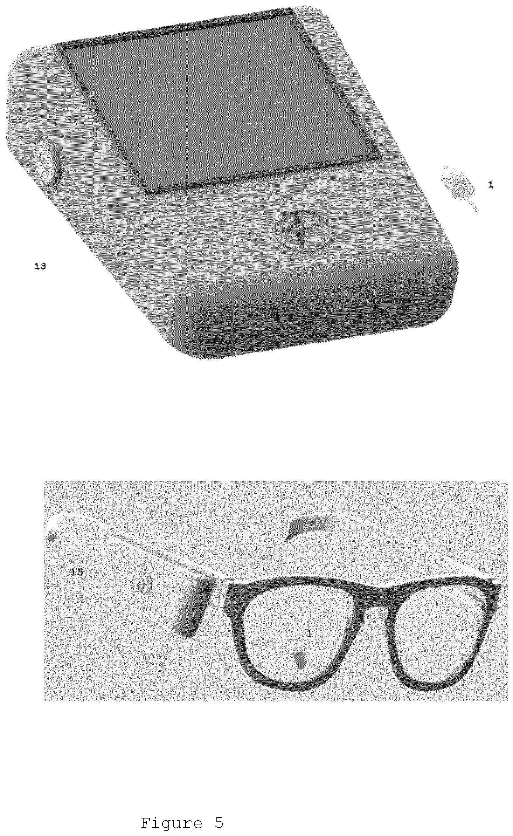

FIG. 5: represents 2 variants of the glaucoma drainage implant system with pressure sensor and valve, and external reading unit, the upper one showing the implant and the

### DETAILED DESCRIPTION OF THE INVENTION

The present glaucoma drainage implant system with pressure sensor and external reading unit consists of the implant device (1) itself, which comprises a microvalve (2) that has a cannula (3) with the corresponding inlet port (4) thereof, where the eye fluid enters when said device (1) is permanently implanted in the eye. Following the cannula is a sensor (5), a flat coil (6), an outlet port (7) through which the ocular fluid drains to reduce intraocular pressure (IOP) in the anterior chamber of the eye, it also comprises a plate (8) to extend the area of absorption by the conjunctiva that covers the microvalve when the device is implanted in the eye; this plate has, at the end opposite the cannula, a fixing hole (9) that helps fixing the implant to the sclera by suture. The pressure sensor, in addition to providing the IOP measurement, serves to monitor the hydraulic resistance. This measure is used to guide the treatment of glaucoma.

The PIO sensor comprises a battery-free capacitive pressure sensor built into the implantable device to provide on-demand IOP measurement.

The microvalve (2) can have a microchip (12) for regulation of an actuator or signal processing and modulation.

The cannula has a microchannel (10) inside, which communicates a microchamber (11) for the sensor (5), the microvalve which is a flange valve and a hole for outlet to the plate (8). The low profile of the implant allows flexibility and compliance with the curvature of the eye.

The shunt reduces the IOP that drains aqueous humor from the anterior chamber of the eye into the subconjunctival space.

The microvalve used is a quasi-bistable microvalve as disclosed in Patent U.S. 8,206,440 consisting of a diaphragm (or cantilever beam or other passive or active structuremechanism to regulate flow) made of a conjugated polymer (or other material) that exhibits high deformability and biocompatibility and whose volume depends on the electrical potential applied by a pair of electrodes, where the sensor and valve actuator are coupled to the drain line, first to deform based on eyeball pressure and second in a buckling position to normally clog the drain line. The sensor is a membrane of conductive polymeric material with those same properties and whose ohmic resistance varies with the mechanical deformation produced by ocular or capacitive pressure or another transduction mechanism.

Preferred materials for implanting include Liquid Crystal Polymer (LCP) or any biocompatible material; in the construction of this implant, the following materials are required (not exclusive):
∘ Materials
▪ LCP sheets with copper
▪ Adhesives for LCP
▪ Gold
▪ Teflon

The present system also includes the External Reading Unit (UEL) (13):
- External Reading Unit (UEL): acquires and processes signals from the PIO sensor and displays the IOP pressure data to be reviewed by medical professionals and based on them make decisions about the patient's condition and initiate changes in medical therapy. The UEL is mainly made up of two parts:

### i) Antenna

The antenna is used to interrogate the PIO sensor. The inventors have implemented a version of the antenna (2.25 cm external diameter, 5 mm internal diameter and 35 µm thick copper), in a rigid plastic housing (13). During the reading, the antenna is placed near the passive sensor between 5 mm and 10 mm distance (or other more remote and shorter distances), and the antenna feeds it by bursts of RF energy. When the sensor is energized, it returns a signal with pressure information. This signal is received by the antenna and sent to the main unit for processing. Other variants of energization such as energy harvesting itself are also included.

The antenna can be included within glasses (14) with data information storage units so that said data can then be processed by the external reading unit (13).

### ii) Main unit

The main unit is the place where all the signals for the patient's electronic system are generated and processed. The custom circuitry generates bursts of RF energy through the antenna that powers the sensor, processes the sensor return signal, and visually displays the PIO pressure information.

The software in the UEL prompts and guides the patient to perform a pressure measurement.

Implant manufacture:
Firstly, the condition of the purchased raw materials is verified to ensure quality as required.

LCP sheets of different thicknesses, generally marketed for the manufacture of flexible printed circuit boards, are copper laminated on both sides. The first processing is to remove said copper from the laminates with ferric chloride solution according to conventional techniques.

Each sheet of LCP is subjected to some chemical process, followed by several steps of conductivity- controlled rinses of the final rinse solution to verify the complete removal of residues that may remain on the surface of the sheets. The sheet that contains the coil, which is made of copper, is subjected to another processing line, keeping the controls in the rinses.

The second step is the cutting of individual sheets according to the stack up design of various sheets. Said cut is made with a Cutter Plotter and adhesive mat to place a stack (stack-up) of "Dry Resist (DR)" paper and foil itself and thus achieve a proper hold of the foil to be cut against the cutting mat. After cutting, the stack-up is removed from the mat and then the resin with its corresponding remover.

The cut in the adhesive sheets of BS (Bonding Sheet) is performed directly on the cutting mat, since these are protected on both sides with PET laminates and do not require removal of adhesives.

Cutting on CuClad Adhesive Sheets is done the same way as LCP Sheets.

Two Gold electrodes deposited by the sputtering technique are used to manufacture the sensor. Furthermore, this technique is also used to obtain selective adhesiveness on certain surfaces of some sheets. To obtain deposited metal patterns, sheets are used in stencils aligned with the previously cut sheets.

Finally, in a previous step to a final thermocompression, a copper electrodeposition is carried out to increase the thickness of certain metal parts and to manufacture a connection path between the pads for the electrodes of the capacitor and the inductor.

The last step is thermocompression of all the individually pre-processed sheets.

For this, the sheets are sequentially placed on heating plates inside a hydraulic press, which have pins for the alignment of all the sheets.

### OPERATION MODE

In one of the operating uses of the proposed system, the implant is implanted by means of surgery in a normal way and in another as a shunt or valves by opening channels in the sclera in the limbus for insertion of the implant cannula and sutures the fixing hole on the back side of the sclera.

The reading units are calibrated by standard tonometry (Goldmann) and control alarms can be set by health professionals.

The user can, whenever he wishes, read the reading unit in the eye at a short distance therefrom (or at a more remote distance by means of suitable telemetry mechanisms) and the eye pressure will be displayed on a screen. If the pressure is above or below the programmed maximum and minimum values, an alarm is activated that alerts the user to take into account on the next visit to the doctor.

Intraocular pressure values are stored and can be exported for future physician analysis during the patient visit.

The microvalve has an active control option where an actuator changes the hydraulic resistance of the microvalve and a microchip controls the actuator.

## Claims

1. Glaucoma drainage implant system with intracular pressure sensors (IOP) and microvalve, and external reading unit **characterized in that** it comprises:
(1) an ocular implant device (1) comprising a main body attached to a cannula (3) with an inlet port (4) which communicates through a microchannel (10) that passes through said cannula with a microchamber of the sensor (11) on which one (or more) sensors (5) is/are located; said microchamber is in fluid communication with a microvalve (2) which regulates the passage of ocular liquid to an outlet port (7), where the microvalve (2) is covered by a plate on said main body; the implant device has also a flat coil (6) to energize the sensor and the microvalve and a microchip (12) for regulation of a microvalve actuator and signal processing and modulation generated by the sensor;
(2) an external reading unit (UEL) (13), which acquires and processes signals from the PIO sensor(s) 5) and displays the PIO pressure data on a screen; the UEL (13) mainly consists of two parts: an antenna and a main unit; wherein the antenna feeds by bursts of RF radio frequency energy to the implant sensor (5) and when the sensor (5) is energized, it returns a signal with information from the IOP, this signal being received by said antenna and sent to the main unit for processing.

2. Glaucoma drainage implant system with intracular pressure sensor (IOP) and microvalve, and external reading unit according to claim 1, **characterized in that** the plate (8) of the implant device (1) has at the opposite end of the cannula (3) a fixation hole (9) that allows the fixation of said implant to the sclera of the eye by suture.
